# EUROPÄISCHE PATENTSCHRIFT

(11) **EP 1 692 126 B1**
(45) Veröffentlichungstag und Bekanntmachung des Hinweises auf die Patenterteilung: **07.11.2012**
(21) Anmeldenummer: 04801220.7
(22) Anmeldetag: 09.12.2004
(51) Int. Cl.: C07D 317/36

(54) **REAKTIVE CYCLISCHE CARBONATE UND HARNSTOFFE ZUR MODIFIZIERUNG VON BIOMOLEKÜLEN, POLYMEREN UND OBERFLÄCHEN**
REACTIVE CYCLIC CARBONATES AND UREAS USED FOR MODIFYING BIOMOLECULES, POLYMERS, AND SURFACES
CARBONATES ET UREES CYCLIQUES REACTIFS SERVANT A MODIFIER DES BIOMOLECULES, POLYMERES ET SURFACES

(30) Priorität: 09.12.2003 EP 03028224
(43) Veröffentlichungstag der Anmeldung: 23.08.2006
(73) Patentinhaber: Henkel AG & Co. KGaA, 40589 Düsseldorf (DE)
(72) Erfinder: KEUL, Helmut, 52074 Aachen (DE); MÖLLER, Martin, 52070 Aachen (DE); PASQUIER, Nicolas, CH-1630 Bulle (CH); UBAGHS, Luc, NL-6224 JP Maastricht (NL)
(86) Internationale Anmeldenummer: PCT/EP2004/014047
(87) Internationale Veröffentlichungsnummer: WO 2005/058863

(56) Entgegenhaltungen:
- WO-A-02/42383
- US-A1- 2002 183 474
- JANSEN, JOHAN F. G. A. ET AL: "Fast Monomers: Factors Affecting the Inherent Reactivity of Acrylate Monomers in Photoinitiated Acrylate Polymerization" MACROMOLECULES , 36(11), 3861-3873 CODEN: MAMOBX; ISSN: 0024-9297, 2003, XP002285157 in der Anmeldung erwähnt

## Beschreibung

Die vorliegende Erfindung betrifft ein Verfahren zur Modifizierung von Substraten, wie Biomolekülen, Polymeren und Oberflächen mit reaktiven cyclischen Carbonaten und Harnstoffen, sowie cyclische Carbonate und Harnstoffe und modifizierte Polymere.

Die dauerhafte Funktionalisierung von Materialoberflächen ist für eine Vielzahl von Materialien und Anwendungen von Bedeutung. Beispielsweise können Materialoberflächen durch Beschichtung mit Heparin biokompatibel gemacht werden. Andere Anwendungen sind schmutzabweisende und bakteriostatische Ausrüstungen und die Verbesserung der Haftung von Klebstoffen und Lacken.

Die Modifikation einer Oberfläche kann entweder reaktiv durch Ausbildung kovalenter Bindungen oder durch Chemisorption bzw. Adsorbtion erfolgen. Beispiele für eine reaktive Modifizierung sind solche mit Isocyanaten, mit Silanen und durch radikalische Pfropfreaktionen. Ein häufig angewandtes Verfahren zum Einführen funktionaler Gruppen an Oberflächen bedient sich der Beschichtung mit funktionalen Silanen, insbesondere Aminosilanen. Die Behandlung mit Aminosilanen ist technisch aufwändig und kann nicht aus wässriger Lösung erfolgen.

Die Chemisorption beruht auf ionischen Wechselwirkungen zwischen negativen Oberflächenladungen und kationischen Verbindungen, z. B. Ammoniumverbindungen. An der Oberfläche fast aller Materialien findet man negativ geladene Gruppen. Bei hydrophoben Polymeren, wie den Polyolefinen, bilden sich negative Oberflächenladungen durch Oxidation an Luft; diese kann künstlich durch eine Plasmabehandlung oder UV-Oxidation verstärkt werden. Polykationische Polymere, wie Polyammoniumverbindungen, adsorbieren auf solchen Oberflächen durch Polyelektrolytkomplexbildung. Durch die Kooperativität der vielfachen Ionenpaarbildung entsteht eine sehr starke Bindung, die selbst bei hohen Ionenstärken und extremen pH-Werten stabil ist.

Auch die gezielte Modifizierung von Biomolekülen ist in vielen Fällen wünschenswert. So kann die biologische Halbwertszeit verschiedener Wirkstoffe beispielsweise durch Anfügen von Polyoxyalkylenresten verbessert werden.

Es besteht ein Bedarf an Reagenzien und Verfahren, die es gestatten, gezielt bestimmte chemisch funktionale Gruppierungen in Biomoleküle und Oberflächen einzuführen. Die beteiligten Umsetzungen sollten unter milden Bedingungen und, soweit Biomoleküle beteiligt sind, ohne deren Denaturierung ablaufen.

Die US-A 5,650,234 beschreibt gemischte Polyethylenglykolcarbonate, die mit Aminogruppen in Aminoglykanen oder Proteinen bzw. Aminogruppen-haltigen Oberflächen glatt reagieren. Die Polyethylenglykolcarbonate gestatten die kovalente Bindung von Biomolekülen an Oberflächen.

Weiterhin besteht ein Bedarf nach leicht zugänglichen polykationischen Verbindungen zur Oberflächenbehandlung, die aus wässriger Lösung aufgebracht werden können und bestimmte funktionale Gruppen enthalten bzw. eine anschließende weitere Modifikation erlauben.

Jansen, I.F.G.A. et al. in Macromolecules 2003, 36, 3861 - 3873, untersuchen die Reaktivität verschiedener Acrylate wie 2-Oxo-1,3-dioxolan-4-yl-methylacrylat. In dieser Publikation wird u.a. die Verbindung 4-[(2-Hydroxyethyl)-aminocarbonyloxymethyl]-2-oxo-1,3-dioxolan genannt.

Die WO 02/42383 beschreibt strahlungshärtbare Zusammensetzungen, die 2-Oxo-1,3-dioxolan-4-yl-methoxycarbonylaminoethylacrylat enthalten können.

Die Erfindung betrifft in einem ersten Aspekt ein Verfahren zur Modifizierung eines Substrates, das über funktionelle Gruppen verfügt, welche unter Hydroxygruppen, primären und sekundären Aminogruppen ausgewählt sind, bei dem man wenigstens ein Substrat unter Bedingungen mit einer Verbindung der Formel I oder II in Kontakt bringt, dass die funktionellen Gruppen unter Öffnung des 1,3-Dioxolanrings bzw. 1,3-Diazaheptanrings und Ausbildung einer kovalenten Bindung mit der Verbindung der Formel I oder II reagieren, 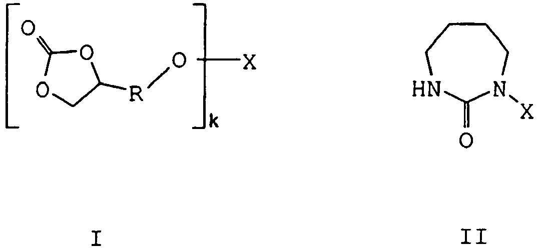 worin
R für C₁-C₁₂-Alkylen steht;
wenn k für 1 steht, X für CO-CH=CH₂, CO-C(CH₃)=CH₂, CO-O-Aryl, C₂-C₆-Alkylen-SO₂-CH=CH₂, oder CO-NH-R¹ steht; und

R¹ für C₁-C₃₀-Alkyl, C₁-C₃₀-Halogenalkyl, C₁-C₃₀-Hydroxyalkyl, C₁-C₆-Alkyloxy-C₁-C₃₀-alkyl, C₁-C₆-Alkylcarbonyloxy-C₁-C₃₀-alkyl, Amino-C₁-C₃₀-alkyl, Mono- oder Di(C₁-C₆-alkyl)amino-C₁-C₃₀-alkyl, Ammonio-C₁-C₃₀-alkyl, Polyoxyalkylen-C₁-C₃₀-alkyl, Polysilo-xanyl-C₁-C₃₀-alkyl, (Meth)acryloyloxy-C₁-C₃₀-alkyl, Sulfono-C₁-C₃₀-alkyl, Phosphono-C₁-C₃₀-alkyl, Di(C₁-C₆-alkyl)phosphono-C₁-C₃₀-alkyl, Phosphonato-C₁-C₃₀-alkyl, Di(C₁-C₆-alkyl)phosphonato-C₁-C₃₀-alkyl oder einen Saccharidrest steht, und
wenn k für eine ganze Zahl von mehr als 1 steht, X steht für (i) den Rest eines Polyamins, an das der in Klammern stehende Formelteil über (CO)NH-Gruppen gebunden ist, oder (ii) ein polymeres Gerüst, an das der in Klammern stehende Formelteil über (CO)-, NH-C₂-C₆-Alkylen-O(CO)- oder (CO)-O- C₂-C₆-Alkylen-O(CO)- Gruppen gebunden ist.

In Formel I steht k für 1 oder eine ganze Zahl von mehr als 1, z. B. 2 oder 3, oder 2 - 10000, wie 10 - 1000..

Bei dem Substrat handelt es sich vorzugsweise um ein Biomolekül, Polymer oder eine Oberfläche.

In einer Ausführungsform des Verfahrens bringt man die Verbindung der Formel I oder II mit einem ersten Substrat unter Bedingungen in Kontakt, dass sich zwischen einem ersten Ende der Verbindung der Formel I oder II und dem ersten Substrat eine kovalente Bindung ausbildet, und bringt das Reaktionsprodukt mit einem zweiten Substrat unter Bedingungen in Kontakt, dass sich zwischen einem zweiten Ende der Verbindung der Formel I oder II and dem zweiten Substrat eine kovalente Bindung ausbildet.

In dieser Ausführungsform umfasst der Rest X im Allgemeinen eine funktionelle Gruppe, die mit dem ersten Substrat reagieren kann, vorzugsweise eine (Meth)acryloyl-, Aryloxy-, Vinylsulfonyl- oder Halogenalkylgruppe.

Das erste und zweite Substrat sind z. B. paarweise eine Oberfläche und ein Biomolekül, ein Biomolekül und ein Modifizierungsmittel oder eine Oberfläche und ein Modifizierungsmittel. Ein Modifizierungsmittel kann eine niedermolekulare oder polymere Verbindung sein, die bestimmte Hydrophilie- oder Lipophilieeigenschaften, Löslichkeit, Stabilität oder dergleichen verleiht, oder ein dirigierendes Molekül.

Die Erfindung betrifft in einem zweiten Aspekt Verbindungen der allgemeinen Formel I oder II 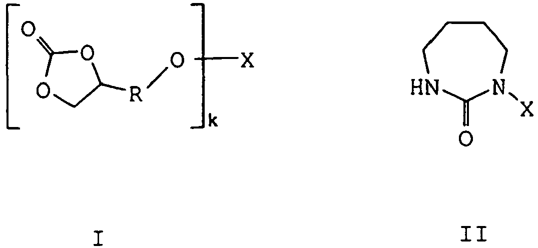 worin
R für C₁-C₁₂-Alkylen, vorzugsweise C₁-C₄-Alkylen, insbesondere Methylen, steht; wenn k für 1 steht, X für C₂ - C₆-Alkylen-SO₂-CH=CH₂ oderCO-NH-R¹ steht; und R¹ für C₁-C₃₀-Alkyl, C₁-C₃₀-Halogenalkyl, C₁-C₃₀-Hydroxyalkyl, C₁-C₆-Alkyloxy-C₁-C₃₀-alkyl, C₁-C₆-Alkylcarbonyloxy-C₁-C₃₀-alkyl, Amino-C₁-C₃₀-alkyl, Mono- oder Di(C₁-C₆-alkyl)amino-C₁-C₃₀-alkyl, Ammonio-C₁-C₃₀-alkyl, Polyoxyalkylen-C₁-C₃₀-alkyl, Polysiloxanyl-C₁-C₃₀-alkyl, Sulfono-C₁-C₃₀-alkyl, Phosphono-C₁-C₃₀-alkyl, Di(C₁-C₆-alkyl)-phosphono-C₁-C₃₀-alkyl, Phosphonato-C₁-C₃₀-alkyl, Di(C₁-C₆-alkyl)phosphonato-C₁-C₃₀-alkyl oder einen Saccharidrest steht, und
wenn R für C₂-C₁₂-Alkylen, insbesondere C₄-Alkylen, steht, X auch für CO-Aryl, CO-CH=CH₂, CO-C(CH₃)=CH₂ oder (Meth)acryloxyloxy-C₁-C₃₀-alkyl-NH-CO stehen kann, ausgenommen 4-[(2-Hydroxyethyl)-aminocarbonyloxymethyl]-2-oxo-1,3-dioxolan.

Wenn k für eine ganze Zahl von mehr als 1 steht, steht X für den Rest eines Polyamins, an das der in Klammern stehende Formelteil über (CO)NH-Gruppen gebunden ist. Beispiele für Polyamine sind Alkylendiamine, Dialkylentriamine, Polydimethylsiloxane mit Aminoalkylgruppen (terminal oder als Seitengruppen), Polyvinylamin, Polyallylamin, Polyethylenimin, Chitosan, Polyamid-Epichlorhydrin-Harze, Polyaminostyrol, Peptide oder Proteine.

Der Begriff "Alkyl" soll geradkettige, verzweigte und cyclische Alkylgruppen mit 1 bis 30, vorzugsweise 1 bis 18, insbesondere 1 bis 12 Kohlenstoffatomen umfassen.

Der Begriff "Aryl" bedeutet vorzugsweise Phenyl oder Naphthyl, das gegebenenfalls mit 1 bis 3 C₁-C₄-Alkyl- oder C₁-C₄-Alkoxygruppen substituiert ist.

Der Begriff "Halogenalkyl" bedeutet eine Alkylgruppe, in der ein oder mehrere oder alle Wasserstoffatome durch Halogen, insbesondere Fluor, ersetzt sind. Der Begriff "Hydroxyalkyl" bedeutet eine Alkylgruppe, in der ein oder mehrere Wasserstoffatome durch Hydroxygruppen ersetzt sind. In gleicher Weise bedeuten die Begriffe "Alkyloxyalkyl", "Alkylcarbonyloxyalkyl", "Aminoalkyl", "Ammonioalkyl", "Polyoxyalkylenalkyl", "Polysiloxanylalkyl", "(Meth)acryloyloxyalkyl", "Sulfonoalkyl", "Phosphonoalkyl" und "Phosphonatoalkyl" einen Alkylrest, in dem ein oder mehrere Wasserstoffatome durch eine Alkyloxygruppe, eine Alkylcarbonyloxygruppe, eine Aminogruppe, eine Ammoniogruppe (NR³₃⁺; worin jedes R³ unabhängig z. B. für C₁-C₁₈-Alkyl oder Benzyl steht), einen Polyoxyalkenylrest, einen Polysiloxanylrest, eine (Meth)acryloyloxygruppe, eine Sulfonsäuregruppe (SO₃H), eine Phosphonsäuregruppe (PO₃H₂) oder Phosphorsäureestergruppe (OPO₃H₂) ersetzt sind. Wenn R¹ für Ammonioalkyl steht, ist die Verbindung von einem Äquivalent eines Anions, vorzugsweise eines physiologisch verträglichen Anions, wie eines Halogenids, z. B. Chlorid oder Bromid, Sulfat, Hydrogensulfat, Methosulfat, Nitrat oder dergleichen begleitet.

Der Polyoxyalkylenrest leitet sich vorzugsweise von Etylenoxid und/oder Propylenoxid ab, insbesondere von Ethylenoxid. Er kann am distalen Ende beispielsweise durch eine Hydroxy-, Alkyloxy- oder Alkaryloxygruppe terminiert sein. Der Polysiloxanylrest leitet sich vorzugsweise von Polydimethylsiloxanen ab.

Bei dem Saccharidrest handelt es sich z. B. um einen Glucosylrest.

Der Begriff "Biömolekül" umfasst alle Moleküle, die aus biologischen Systemen isoliert sind und/oder mit biologischen Systemen oder Teilen davon in Wechselwirkung treten können. Hierzu zählen vor allem Peptide, Proteine, Proteoglykane, Enzyme, Markierungsstoffe, Antikörper, Rezeptormoleküle, Antigene, Wirkstoffe. Spezielle Beispiele sind Heparin, Gewebeplasminogenaktivator, Streptokinase, Prostaglandine und dergleichen.

In bevorzugten Ausführungsformen steht R¹ für
- (CH₂)ₙ-CH₃,
- (CH₂)ₙ-(CF₂)ₘ-CF₃,
- (CH₂)ₙ-[Si(CH₃)₂-O]ₚ-H,
- (CH₂)ₙ-Si(OSi(CH₃)₃)₃,
- (CH₂)ₙ-(O-CH₂-CHR⁴)ₚ-OR³,
- R²-OH,
- R²-NH₂,
- R²-NR³₃⁺ Y⁻,
- R²-SO₃H,
- R²-PO₃H₂,
- R²-OPO₃H₂
oder einen Saccharidrest,
wobei R² für C₁-C₁₈-Alkylen, R³ für C₁-C₁₈-Alkyl oder Benzyl und R⁴ für Wasserstoff oder Methyl steht,
Y für ein Äquivalent eines Anions, wie den oben genannten, steht,
n und m unabhängig voneinander für eine ganze Zahl von 0 bis 12 stehen; und
p für eine ganze Zahl von 1 bis 100, vorzugsweise 2 bis 50, steht.

Die Verbindungen können auf verschiedene Weise unter Anwendung von Standardverfahren der organischen Synthese hergestellt werden. Bevorzugte Herstellungsarten sind in den Beispielen veranschaulicht.

Verbindungen der Formel I, worin X für (Meth)acryloyl steht, können durch Umsetzen von 2-Oxo-1,3-dioxolan-4-yl-alkanolen mit (Meth)acrylsäurechlorid erhalten werden; Verbindungen der Formel I, worin X für -CH₂-CH₂-SO₂-CH=CH₂ steht, durch Umsetzen von 2-Oxo-1,3-dioxolan-4-yl-alkanolen mit Divinylsulfon erhalten werden.

Verbindungen der Formel I, in denen X für ein polymeres Gerüst steht, können durch radikalische Polymerisation oder kontrollierte radikalische Polymerisation, z. B. durch ATRP (Atom Transfer Radical Polymerization), von Verbindungen erhalten werden, worin X eine (Meth)acryloylgruppe umfasst. Dabei werden vorzugsweise Comonomere mitverwendet, wie C₁-C₁₂-Alkyl(meth)acrylate, insbesondere Methyl(meth)acrylat, und/oder Vinylaromaten, wie Styrol. In einer besonderen Ausführungsform werden außerdem Comonomere mitverwendet, die über gegenüber Nuclephilen reaktive Seitengruppen verfügen und deren Reaktivität verschieden ist von der des 2-Oxo-1,3-dioxolanyl-Restes. Ein bevorzugtes reaktives Comonomer ist 2-Phenoxycarbonyloxy-ethyl(meth)acrylat. Eine bevorzugte Verbindung der Formel I, worin X für ein polymeres Gerüst steht, ist ein Copolymer von 2-Oxo-1,3-dioxolan-4-yl-methyl(meth)acrylat, 2-Phenoxycarbonyloxy-ethyl(meth)acrylat und Methyl(meth)acrylat.

Die obigen Verbindungen reagieren unter milden Bedingungen mit Nukleophilen, z. B. Hydroxy- oder Aminogruppen von Biomolekülen oder Polymeren oder an Substratoberflächen, unter Ringöffnung und Ausbildung einer kovalenten Bindung, wie im nachstehenden Schema veranschaulicht ist (worin D für das Nukleophil steht und R' für den Rest des Biomoleküls, Polymers bzw. der Substratoberfläche steht; verschiedene Nukleophile D sind in der Tabelle 2 der nachstehenden Beispiele veranschaulicht):

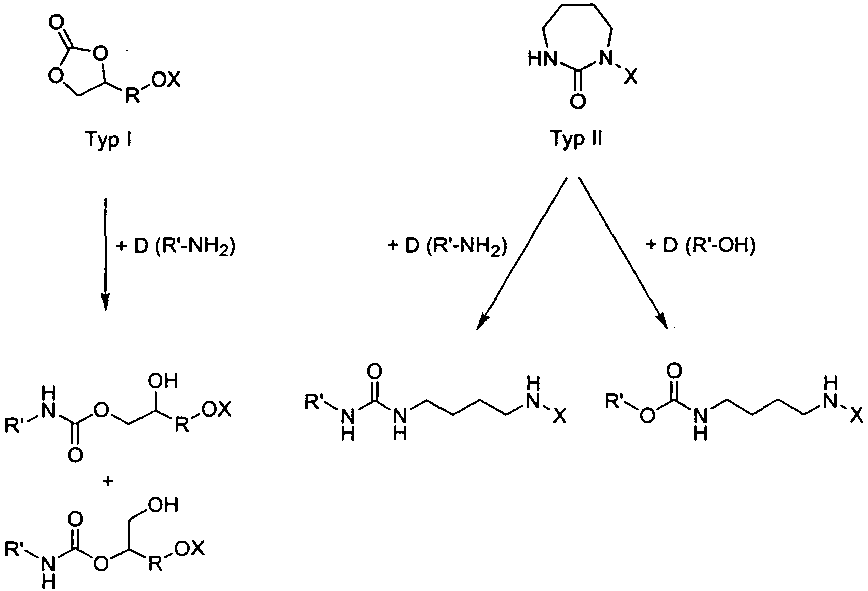

Verbindungen der Formel I reagieren dabei bevorzugt mit primären oder sekundären Aminogruppen; Verbindungen der Formel II bevorzugt mit primären oder sekundären Aminogruppen und Hydroxygruppen.

Auf diese Weise gestatten die erfindungsgemäßen Verbindungen, eine große Bandbreite verschiedener Reste X in Biomoleküle oder Polymere einzuführen bzw. an Substratoberflächen zu binden.

Oberflächen, die nach diesem Verfahren behandelt werden können, sind z. B. die Oberflächen von Materialien, die intrinsische Amino- und/oder Hydroxygruppen aufweisen, oder Oberflächen, die nach an sich bekannten Verfahren mit Aminosilanen behandelt wurden.

Ein weiterer Gegenstand der Erfindung sind modifizierte Polymere, d. h. Umsetzungsprodukte der erfindungsgemäßen Verbindungen mit einem Polymer, das über funktionelle Gruppen verfügt, die unter Hydroxygruppen, primären und sekundären Aminogruppen ausgewählt sind. Die Umsetzung der erfindungsgemäßen Verbindungen mit einem Polyamin oder einem Polyol erlaubt die Einführung der funktionalen Gruppe X in die Polymerstruktur. Beispiele für Polyamine sind Polyvinylamin, Polyallylamin, Polyethylenimin, Chitosan, Polyamid-Epichlorhydrin-Harze, wie sie unter der Bezeichnung Hercosett® vertrieben werden, Polyaminostyrol, Peptide oder Proteine, wie Gelatine. Bevorzugte Proteine sind modifizierte Keratinpolypeptide, die durch reverse Proteolyse (Plastein-Reaktion) mit Lysin (2,6-Diaminohexansäure) angereichert sind.

Bei der Umsetzung des Polymers mit den erfindungsgemäßen Verbindungen handelt es sich um eine polymeranaloge Reaktion. Die Konzentration der funktionellen Gruppen X im Umsetzungsprodukt lässt sich durch Wahl des stöchiometrischen Verhältnisses der Amino- oder Hydroxygruppen im Polymer zum Reagenz I oder II einstellen.

In bevorzugten Umsetzungsprodukten steht zumindest ein Teil der Reste R¹ für Ammonioalkyl, d. h. es handelt sich bei den Umsetzungsprodukten um kationische Polylelektrolyte. Die erhaltenen kationischen Polylelektrolyte können auf einer anionischen Materialoberfläche durch Polyelektrolytwechselwirkung dauerhaft haften.

Bevorzugt wird die Umsetzung so ausgeführt, dass bei der Umsetzung mit den erfindungsgemäßen Verbindungen nicht alle Hydroxy- und/oder Aminogruppen des Polymers teilnehmen, so dass die Umsetzungsprodukte noch reaktive Hydroxy- und/oder primäre oder sekundäre Aminfunktionen enthalten. Auf diese Weise lassen sich reaktive NH₂-, NH- bzw. OH-Gruppen in die Oberflächen einführen, die eine schnelle und effektive Anbindung unterschiedlicher Beschichtungen erlauben. Beispiele sind Epoxide, Isocyanate, Anhydride und Carbonsäuren sowie Acrylate (Michael-Addition) aber auch Wirkstoffe jeder Art können auf diese Weise auf der Oberfläche immobilisiert werden.

In weiteren bevorzugten Umsetzungsprodukten steht ein Teil der Reste R¹ für Ammonioalkyl, und ein Teil der Reste R¹ für einen oder mehrere davon verschiedene Reste. Das Umsetzungsprodukt besitzt neben den kationischen Gruppen, die der Anbindung/Haftung auf einer Materialoberfläche dienen, weitere funktionelle Gruppen. Diese können genutzt werden, um unterschiedliche funktionelle Gruppen auf einer Oberfläche zu verankern. Dieses können Biomoleküle, wie Wirkstoffe, z. B. Bakterizide, Insektizide oder Medikamente sein, die gegebenenfalls erst nach hydrolytischen oder enzymatischen Abbau durch Freisetzung aktiv werden.

Die Erfindung betrifft in einem weiteren Aspekt ein Verfahren zur Modifizierung von Oberflächen, bei dem man die Oberfläche mit einem vorstehend beschriebenen Umsetzungsprodukt, insbesondere einem solchen, in dem zumindest ein Teil der Reste R¹ für Ammonioalkyl steht, in Kontakt bringt. Bei den Oberflächen, die auf diese Weise behandelt werden können, kann es sich um solche aus beliebigen Materialien handeln, z. B. Glas, keramische Werkstoffe, Metalle, Kunststoffe, wie Polyolefine, Polystyrole, schlagzäh modifizierte Polystyrole. Es können auch die Oberflächen von Fasern oder Filamenten behandelt werden. Es ist ein besonderer Vorteil der Erfindung, dass die Beschichtung oder die Applikation des Umsetzungsprodukts aus einer wässrigen Lösung erfolgen kann und keiner komplizierten Vorbereitung und Ausrüstung bedarf wie beispielsweise die Oberflächenbehandlung mit Aminosilanen.

Neben der direkten Oberflächenbeschichtung auch als Schlichten und Avivagen von Fasern ergeben sich für die so dargestellten Polymere Anwendungen als Dispergatoren und (reaktive) Emulgatoren. Weitere Anwendungen im Bereich der polymeren Additive betreffen Haftvermittler, Klebstoffe, Haftklebstoffe und die Immobilisierung von Wirkstoffen.

Die Erfindung wird durch die beigefügte Figur und die nachstehenden Beispiele näher veranschaulicht.

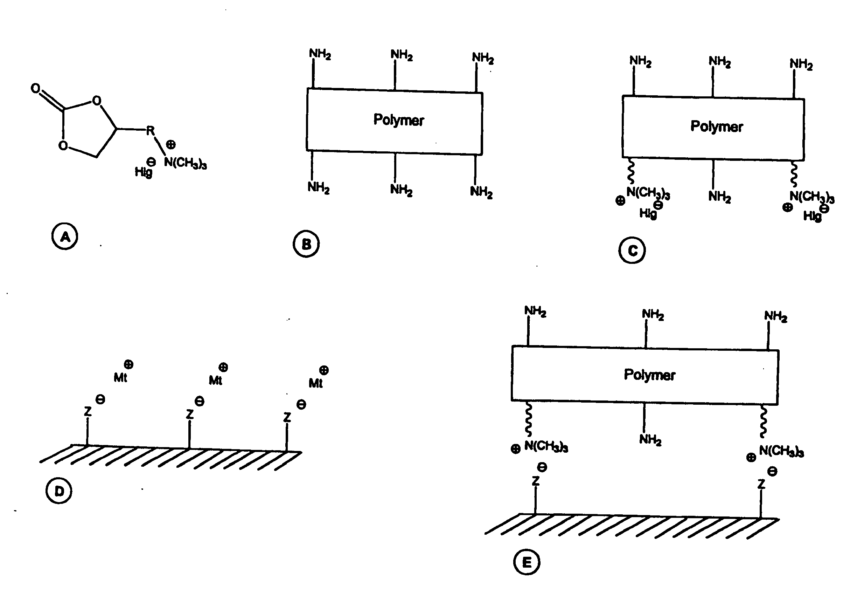

Fig. 1 zeigt schematisch die Herstellung einer erfindungsgemäß modifizierten polykationischen Materialoberfläche. (A) bezeichnet die eingesetzte erfindungsgemäße Verbindung der Formel I (worin R z. B. für -CH₂-OCONH-(CH₂)₃- und Hlg⁻ für ein Halogenid steht) mit einem Ammonium-Substituenten. Die Umsetzung mit dem Polyamin (B) führt zum Umsetzungsprodukt (C). (D) zeigt eine Materialoberfläche mit negativen Oberflächenladungen Z⁻, die locker mit Kationen Mt⁺ assoziiert sind. Bei Behandlung der Materialoberfläche (D) mit dem Umsetzungsprodukt (C) wird das Salz Mt⁺ Hlg⁻ verdrängt und es bildet sich aufgrund der vielfachen Ionenpaarwechselwirkung eine starke Haftung des Polymers zur Oberfläche aus. Am Polymer stehen Aminogruppen (-NH₂) für weitere Modifizierungen zur Verfügung.

### Beispiel 1:

### Verbindung A.1

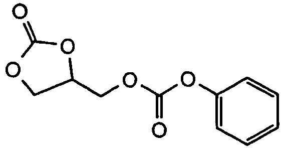

Die Verbindung kann nach der von J. F. G. A. Jansen, A. A. Dias, M. Dorschu, B. Coussens, Macromolecules 2003, 36, 3861-3873 beschriebenen Vorschrift erhalten werden.

### Verbindung A.2

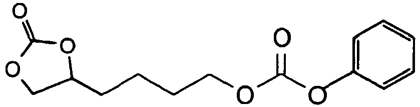

In einem mit Magnetrührer, Tropftrichter und Rückflusskühler versehenen Dreihalskolben werden 27 g (202 mmol) 1,2,6- Hexantriol in 65 mL 1,4-Dioxan gelöst. Bei 0° C werden 126,5 g; 101,7 mL (808 mmol; 4,0 Eq.) Chlorameisensäurephenylester im Zeitraum von ca. 1 h zugetropft und anschließend eine Lösung aus 44 mL (313 mmol) Triethylamin in 200 mL Toluol über eine Zeitraum von 3 h langsam zugetropft. Nach beendeter Zugabe wird zur Reaktionsvervollständigung 16 h bei Raumtemperatur gerührt. Im Anschluss werden zur nun trüben Lösung 68 mL 1 M HCl-Lösung zugetropft und die Reaktionslösung mit ca. 300 mL Ethylacetat aufgenommen. Die organische Phase wird nacheinander mit 1 M HCl-Lösung und H₂O gewaschen, über Na₂SO₄ getrocknet, das Trockenmittel abfiltriert und das Lösungsmittel abdestilliert. Zurück bleibt ein gelbliches, nach Phenol riechendes Öl. Das Phenol wird mittels Hochvakuum-Kondensation bei 70° C entfernt. Das als Nebenprodukt entstehende Diphenylcarbonat wird mittels Soxlettextraktion aus dem braungelben Öl extrahiert. Dazu wird das Öl in 3-fache Menge Kieselgel aufgenommen und in einer Soxletthülse gefüllt 16 h mit Pentan extrahiert. Das Produkt wird durch Behandlung des Kieselgels mit Ethylacetat gelöst und das hellgelbe, hochvisköse Öl im HV unter Rühren für 16 h getrocknet.Ausbeute: 32 g (114 mmol; 56 % d. Theorie)
¹H-NMR (300 Mhz, DMSO-d₆):
δ_{H} = 1,46 (m, 2H, CH₂-5); 1,71 (m, 4H, CH₂-4, CH₂-6); 4,14 (d/d, 1H, CH₂-2^{a}, ³*J* = 8,3 Hz, ³*J*= 7,9 Hz); 4,22 (t, 2H, CH₂-7*,* ³*J* = 6,4 Hz); 4,58 (t, 1H, CH₂-2^{b}, ³*J* = 8,3 Hz); 4,79 (qu, 1H, CH-3, ³*J* = 7,2 Hz); 7,25 (d, 2H, CH-10, ³*J* = 7,2 Hz); 7,29 (d, 1H, CH-12, ³*J* = 7,5 Hz); 7,44 (d/d, 2H, CH-11, ³*J* = 7,9 Hz, ³*J* = 7,5 Hz) ppm.
¹³C- NMR- Spektrum (75 MHz, DMSO-d₆):
   δ_{C} = 20,39 (C-5, 1 C); 27,55 (C-6, 1C); 32,33 (C-4, 1C); 68,16 (C-7,1C); 69,12 (C-2,1C); 76,85 (C-3, 1C); 121,12 (C-10, 2C); 126,06 (C-12, 1C); 129,55 (C-11, 2C); 150,74 (C-9, 1C); 153,06 (C-8, 1C); 154,87 (C-1, 1C) ppm.

### Verbindung B (2-Oxo-[1,3]diazepan-1-kohlensäurephenylester)

Die Verbindung wurde wie folgt hergestellt: Zu einer Lösung von Tetramethylenharnstoff (1 Äquivalent) in Dichlormethan wurden zunächst 1,5 Äquivalente Triethylamin und danach bei 0 bis 50 °C langsam Phenylchloroformiat (1,5 Äquivalente) zugegeben. Nach beendeter Zugabe rührte man weitere 30 min. bis 2 h. Danach wurde abgekühlt, der Niederschlag (Amin-Hydrochlorid) abfiltriert und mit wenig Lösungsmittel gewaschen. Das Produkt wurde durch Säulenchromatographie gereinigt (Laufmittel: Diethylether / Essigsäureethylester =1/1) und bei Raumtemperatur im Vakuum (10⁻² mbar) getrocknet. Ausbeute: 90% d. Th.

Die Herstellung weiterer Verbindungen erfolgt z. B. nach folgendem Schema:

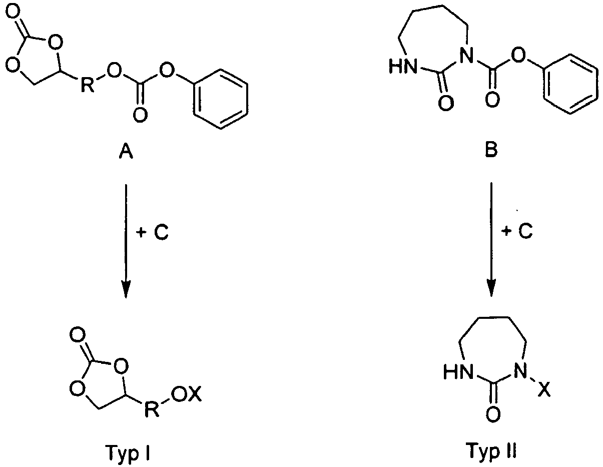

In einem Kolben geeigneter Größe, der mit Rückflusskühler und regelbarem Rührer versehen ist, gibt man eine der in Tabelle 1 angegebenen Komponenten C in der angegebenen Menge (in Äquivalenten) bei der angegebenen Temperatur unter Rühren zu einer vorgelegten Lösung der Komponente A.1 oder A.2 (zur Herstellung weiterer Verbindungen der Formel I) bzw. Komponente B (zur Herstellung weiterer Verbindungen der Formel II) in einem geeigneten Lösungsmittel (z. B. Chloroform, Dichlormethan, Essigsäureethylester, Dimethylsulfoxid, Dimethylacetamid, Dimethylformamid). Anschließend wird die Lösung über die in Tabelle 1 angegebene Zeit gerührt. Das Produkt wird nach üblichen Verfahren isoliert (Abdestillieren der flüchtigen Bestandteile, Ausfällen und/oder Chromatographie).

**Tabelle 1**

| Nr. | Komponente C | Herstellung von Typ I | | | Herstellung von Typ II | | |
|---|---|---|---|---|---|---|---|
| | | Äq. | Temp. [°C] | Zeit [h] | Äq. | Temp. [°C] | Zeit [h] |
| 1 | Alkylamin | 1,0 - 1,5 | (-10) - 50 | 1 - 48 | 1-5 | 0 - 100 | 1 - 48 |
| 2 | Fluoralkylamin | 1,0 -1,5 | (-10) - 50 | 1 - 48 | 1 - 5 | 0 - 100 | 1 - 48 |
| 3 | Aminopolysiloxan | 1,0 - 1,5 | (-10) - 50 | 1 - 48 | 1-5 | 0-100 | 1 - 48 |
| 4 | Polyoxyethylen monoamin | 1,0 - 1,5 | (-10) - 50 | 1 - 48 | 1-5 | 0 - 100 | 1 - 48 |
| 5 | α-Aminoalkyl-ω-alkoxypolyethylenglycol | 1,0 - 1,5 | (-10) - 50 | 1 - 48 | 1-5 | 0 - 100 | 1 - 48 |
| 6 | Aminoalkohol | 1,0 - 1,5 | (-10) - 50 | 1 - 48 | 1-5 | 0 - 100 | 1 - 48 |
| 7 | Diamin | - | - | - | 5-10 | 0 - 100 | 1 - 48 |
| 8 | (Alkyl)acrylsäure aminoalkylester | 1,0 - 1,5 | (-10) - 50 | 1 - 48 | 1-5 | 0 - 100 | 1 - 48 |
| 9 | Aminomonosaccharid (Glucosamin) | 1,0- 1,5 | (-10) - 50 | 1-48 | 1-5 | 0 - 100 | 1 - 48 |
| 10 | Aminoalkylphosphonsäure | 1,0 - 1,5 | (-10) - 50 | 1 - 48 | 1-5 | 0 - 100 | 1 - 48 |
| 11 | Phosphorsäure monoaminoalkylester | 1,0- 1,5 | (-10) - 50 | 1 - 48 | 1-5 | 0 - 100 | 1 - 48 |
| 12 | Aminoalkansulfonsäure | 1,0- 1,5 | (-10) - 50 | 1 - 48 | 1-5 | 0 - 100 | 1 - 48 |
| 13 | Aminoalkyl-trialkylammoniumsalz | 1,0- 1,5 | (-10) - 50 | 1 - 48 | 1-5 | 0 - 100 | 1 - 48 |

### Verbindung I.1 (R = CH₂, X = CO-NH-(CH₂)₃-N(CH₃)₂

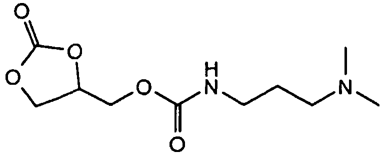

In einem mit Innenthermometer und Tropftrichter versehenen Zweihalskolben werden 10 g (42 mmol) **A.1** in 100 mL THF gelöst. Bei 0° C wird eine Lösung aus 4 g (40 mmol) 3,3-Dimethylaminopropylamin in 50 mL THF so langsam zugegeben, dass die Temperatur 5° C nicht überschritten wird. Zur Reaktionsvervollständigung wird die Reaktionslösung für 16 h weiter gerührt. Dabei lässt man die Temperatur langsam bis Raumtemperatur steigen. Der Reaktionsansatz wird im Rotationsverdampfer eingeengt. Zur Entfernung von Phenol wird der Rückstand mit 200 mL einer Mischung aus THF/Et₂O (1:1) aufgenommen und diese 5-mal mit je 50 mL H₂O extrahiert. Die vereinigten wässrigen Phasen werden 5-mal mit je 70 mL CHCl₃ extrahiert. Die organische Phase wird über Na₂SO₄ getrocknet, das Trockenmittel wird abfiltriert und das Lösungsmittel mittels Rotationsverdampfer abdestilliert. Das farblose, hochvisköse Öl wird unter Rühren im HV über Nacht getrocknet. Ausbeute: 5,1 g (21 mmol; 50 %) ¹H-NMR (300 MHz, CDCl₃):
δ_{H} = 1,67 (qu, 2H, CH₂-7, ³*J* = 6,8 Hz); 2,22 (s, 6H, CH₃-9); 2,35 (t, 2H, CH₂-8, ³*J* = 6,8 Hz); 3,24 (q, 2H, CH₂-6, ³*J* =6,0 Hz), 4,30 (m, 2H, CH₂-4); 4, 36 (d/d, 1H, CH₂-2^{a}, ³*J* = 8,7 Hz, ³*J* = 6,0 Hz); 4,55 (t, 1H, CH₂^{b}-2, ³*J* = 8,3 Hz); 4,92 (qu, 1H, CH-3, ³*J* = 7,2 Hz); 6,2 (s, br., 1H, NH) ppm.
¹³C- NMR- Spektrum (75 MHz, CDCl₃):
   δ_{c} = 26,69 (C-7, 1 C); 40,52 (C-6, 1 C); 45,34 (C-9, 2C); 57,90 (C-8, 1 C); 63,24 (C-4, 1 C); 66,04 (C-2, 1 C); 74,55 (C-3, 1 C); 154,78 (C-1, 1 C); 155,68 (C-5, 1 C) ppm.

### Verbindung 1.2 (R = CH₂, X = CO-NH-(CH₂)₂-N(CH₃)₂)

Ausgehend von 20.1 mmol **A.1** und 23 mmol Dimethylaminoethylamin wurde das Produkt in 63%-iger Ausbeute erhalten.

### Verbindung 1.3 (R = CH₂, X = CO-NH-(CH₂)₃-CH₃)

Ausgehend von 20.1 mmol **A.1** und 23 mmol Butylamin wurde das Produkt in 80%-iger Ausbeute erhalten.

### Verbindung 1.4 (R = CH₂, X = CO-NH-(CH₂)₁₁-CH₃)

In einem mit Innenhermometer und einem Tropftrichter ausgestatteten Zweihalskolben wurde Dicarbonat **A.1** (2.00 g, 8.40 mmol) in 20 mL trockenem THF gelöst. Nach Kühlung auf 0 °C wurde eine Suspension aus Dodecylamin (1.56 g, 8.40 mmol) in 10 mL trocknem THF langsam zugegeben, und die Temperatur unter 5 °C gehalten und über Nacht auf RT erwärmen gelassen. Das Lösungsmittel wurde unter Vakuum (-20 mbar, 40 °C) entfernt. Das mit einer C₁₂-Alkylkette funktionalisierte zyklische Carbonat wurde in 15 mL CHCl₃ gelöst und mittels Zugabe von 30 mL Et₂O kristallisiert. (Ausbeute: 93 %).
¹H-NMR (300 MHz, CDCl₃, TMS):
δ_{H} = 0.88 (t, 3H, C*H*₃-17), 1.19-1.36 (s, 18H, C*H*₂-(8-16)), 1.41-1.56 (m, 2H, C*H*₂-7), 3.16 (d×t, 2H, C*H*₂-6), 4.22-4.38 (m, 3H, C*H*^{a}H^{b}-2, C*H*₂-4), 4.55 (d×d, 1H, CH^{a}*H*^{b}-2), 4.86-4.95 (m, 1H, C*H*-3), 5.08-5.18 (b, 1H, N*H*) ppm.
¹³C-NMR (75 MHz, CDCl₃, TMS):
   δ_{C} = 14.1 (C-17), 22.7 (C-16), 26.7 (C-8), 29.3-29.8 (7C, C-(9-15)), 31.9 (C-7), 41.3 (C-6), 63.3 (C-4), 66.0 (C-2), 74.5 (C-3), 154.8 (C-1), 155.6 (C-5) ppm.

### Verbindung 1.5 (R = CH₂, X = CO-NH-(CH₂)₁₇-CH₃)

In einem mit innerem Thermometer und einem Tropftrichter ausgestatteten Zweihalskolben wurde Dicarbonat **A.1** (5.00 g, 21.0 mmol) in 20 mL trocknem THF gelöst und auf 0 °C gekühlt. Eine Suspension aus Stearylamin (5.65 g, 21.0 mmol) in 20 mL trocknem THF wurde langsam zugegeben, und die Temperatur unter 5 °C gehalten. Die Reaktion wurde für 2 Stunden auf 0 °C und 1.5 Tag auf RT gerührt. Das Lösungsmittel wurde unter Vakuum (∼20 mbar, 40 °C) entfernt. Das mit einer C₁₈-Alkylkette funktionalisierte zyklische Carbonat wurde aus CHCl₃ umkristallisiert. (Ausbeute: 85 %).

### Verbindung 1.6 (R = CH₂, X = CO-NH-(CH₂)₃-N(CH₃)₃I)

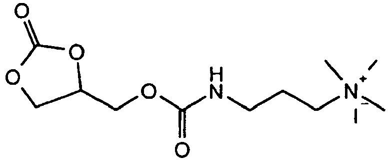

Zu einer Lösung von 0,50 g (2 mmol) Verbindung **I.1** in 5 mL THF werden eine Lösung aus 0,70 g (5 mmol) CH₃I in 5 mL THF schnell zugetropft. Es fällt ein farbloser Niederschlag während des einstündigen Rührens aus. Die THF-Phase wird abdekantiert und der Niederschlag mehrmals mit THF gewaschen. Das erhaltene Produkt wird im HV über Nacht getrocknet. Ausbeute: 0,77 g (1,98 mmol; 99% d. Theorie) ¹H-NMR (300 Mhz, DMSO-d₆):
δ_{H} = 1,85 (m, 2H, CH₂-7); 3,08 (s, 9H, CH₃-9); 3,33 (m, 4H, CH₂-8, CH₂-6); 4,17-4,31 (m, 3H, CH₂-4, CH₂-2^{a}); 4,57 (t, 1H, CH₂-2^{b}, ³*J* = 8,7 Hz); 5,04 (m, 1H, CH-3); 7,48 (t, 1H, NH) ppm.
¹³C- NMR- Spektrum (75 MHz, DMSO-d₆):
   δ_{C} = 22,97 (C-7, 1C); 37,42 (C-6, 1 C); (C-9, 1 C); 52,26 (C-8, 1 C); 63,29 (C-4, 1C); 65,95 (C-2, 1C); 74,75 (C-3, 1 C); 154,72 (C-1, 1C); 155,68 (C-5, 1C) ppm.

### Verbindung 1.7 (R = (CH₂)₄, X = CO-NH-(CH₂)₃-N(CH₃)₂)

Die Verbindung wurde analog zur Verbindung **I.1** hergestellt, wobei man die Verbindung **A.2** an Stelle der Verbindung **A.1** als Ausgangsmaterial verwendete.

### Verbindung 1.8 (R = (CH₂)₄, X = CO-NH-(CH₂)₅-CH₃)

Die Verbindung wurde analog zur Verbindung **I.3** hergestellt, wobei man die Verbindung **A.2** an Stelle der Verbindung **A.1** und Hexylamin an Stelle von Butylamin als Ausgangsmaterialien verwendete.

### Verbindung I.9 (R = (CH₂)₄, X = CO-NH-(CH₂)₁₁-CH₃)

Die Verbindung wurde analog zur Verbindung **I.4** hergestellt, wobei man die Verbindung **A.2** an Stelle der Verbindung **A.1** als Ausgangsmaterial verwendete.

### Verbindung I.10 (R = CH₂, X = CO-NH-(CH₂)₂-N(CH₃)₃ ½ (SO₄))

Die Verbindung wurde durch Umsetzung von 14.7 mmol der Verbindung **1.2** mit 29 mmol Dimethylsulfat in 90%-iger Ausbeute erhalten.

### Verbindung I.11 (R = (CH₂)₄, X = CO-NH-(CH₂)₃-N(CH₃)₃I)

Die Verbindung wurde analog zur Verbindung **1.6** hergestellt, wobei man die Verbindung **1.7** an Stelle der Verbindung **I.1** als Ausgangsmaterial verwendete.

### Verbindung I.12 (R = CH₂, X = CO-NH-(CH₂)₃-(Si(CH₃)₂-O)₈-Si-(CH₂)₃-NH-CO)

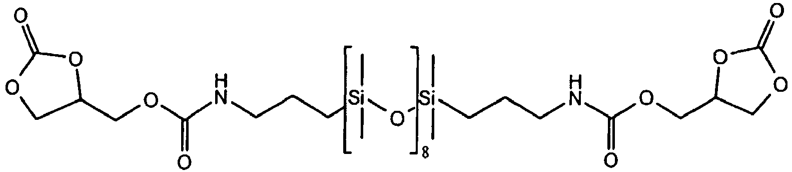

Zu einer Lösung aus 3 g (13 mmol) **A.1** in 30 mL THF wird eine Lösung aus 4,6 g (4 mmol) Nonadimethylsiloxandipropylamin in 50 mL THF bei 0° C langsam zugetropft. Zur Vervollständigung der Reaktion wird die Lösung für 48 h weiter gerührt. Dabei lässt man die Temperatur langsam bis zur Raumtemperatur steigen. Der Reaktionsansatz wird im Rotationsverdampfer eingeengt. Zurück bleibt neben dem Produkt, überschüssiges Ausgangsprodukt und Phenol. Die Verunreinigungen werden durch Lösen in Pentan entfernt. Der Umsatz an Nonadimethylsiloxandipropylamin ist quantitativ.

¹H-NMR (300 Mhz, CDCl₃):
δ_{H} = 0,03 (s, 6H, CH₃-10); 0,07 (s, 48H, CH₃-9); 0,53 (t, 4H, CH₂-8, ³*J* = 8,1 Hz); 1,54 (m, 4H, CH₂-7); 3,16 (m, 4H, CH₂-6); 4,30 (m, 3H, CH₂-4, CH₂-2^{a}); 4,54 (t, 1H, CH₂-2^{b}, ³*J* = 8,7 Hz); 4,90 (m, 2H, CH-3).

### Verbindung I.13 (R = (CH₂)₄, X = CO-NH-(CH₂)₃-Si(CH₃)₂-O)₈-Si-(CH₂)₃-NH-CO)

Die Verbindung wurde analog zur Verbindung **I.12** hergestellt, wobei man die Verbindung **A.2** an Stelle der Verbindung **A.1** als Ausgangsmaterial verwendete.

### Verbindung 1.14 (R = CH₂, X = CO-NH-(CH₂)₃-Si(OSiMe₃)₃)

Zu einer Lösung aus 3 g (12,6 mmol) **A.1** in 30 mL THF wird eine Lösung aus 4,41 g (12,5 mmol) Tris(trimethylsiloxy)silanpropylamin in 50 mL THF bei 0° C langsam zugetropft. Zur Vervollständigung der Reaktion wird die Reaktionslösung für 16 h weiter gerührt. Dabei lässt man die Temperatur langsam bis zur Raumtemperatur steigen. Der Reaktionsansatz wird im Rotationsverdampfer eingeengt. Ausbeute: quantitativ.

### Verbindung 1.15 (R = CH₂, X = CO-NH-[CH(CH₃)CH₂O]ₙ(CH₂CH₂O])ₙ-CH₃)

Zu einer Lösung von 0,5 g (2,1 mmol) **A.1** in 5 mL THF wird eine Lösung aus 2,27 g (2,1 mmol) Jeffamin M-1000 in 25 mL THF bei 0° C langsam zugetropft. Zur Reaktionsvervollständigung wird die Reaktionslösung für 72 h weiter gerührt. Dabei lässt man die Temperatur langsam bis zur Raumtemperatur steigen. Der Reaktionsansatz wird im Rotationsverdampfer eingeengt. Ausbeute: quantitativ

### Verbindung 1.16 (R = CH₂, X = CH₂CH₂SO₂CH=CH)

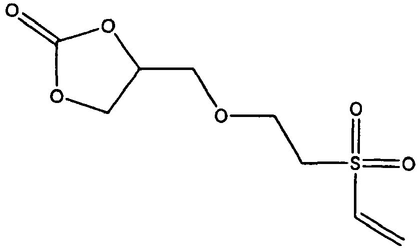

Zu einer Lösung von 4-(Hydroxymethyl)-[1,3]dioxolan-2-on (0.2 g, 1.63 mmol) in Tetrahydrofuran (5 mL) wurden Diazabicycloundecan (25 µL) und Divinylsulfon (0.99g, 8.40 mmol) zugegeben und 24 h bei Raumtemperatur unter einer Stickstoffatmosphäre gerührt. Zur Reinigung wird Tetrahydrofuran abdestilliert, der Rückstand in Pentan gelöst und durch Kühlen auf 0°C das Produkt als viskoses Öl abgetrennt. Die Ausbeute beträgt 75%.

### Beispiel 2: Anwendungsbeispiele für Verbindungen des Typs I und Typs II

In einem Kolben geeigneter Größe, der mit Rückflusskühler und regelbarem Rührer versehen ist, gibt man eine der in Tabelle 2 angegebenen Komponenten D, die einer der Komponenten C entspricht oder ein stickstoffhaltiges Polymer oder ein Polyol ist, in der angegebenen Menge (in Äquivalenten, bezogen auf die Zahl der funktionellen Gruppen im Polymer) bei der angegebenen Temperatur unter Rühren zu einer Lösung einer Verbindung der Formel I oder II in einem geeigneten Lösungsmittel (z. B. Wasser, Chloroform, Dichlormethan, Essigsäureethylester, Dimethylsulfoxid, Dimethylacetamid, Dimethylformamid). Anschließend wird die Lösung über die in Tabelle 2 angegebene Zeit gerührt. Das Produkt wird nach üblichen Verfahren isoliert, z. B. durch Waschen, Abdestillieren der flüchtigen Bestandteile, Ausfällen, Chromatographie.

**Tabelle 2**

| Nr. | Komponente D | Verbindung I | | | Verbindung II | | |
|---|---|---|---|---|---|---|---|
| | | Äq. | Temp. [°C] | Zeit [h] | Äq. | Temp. [°C] | Zeit [h] |
| 1 | Komponente C | 0,1 - 5 | 20 - 100 | 1 - 48 | 0,1 - 5 | 75 - 150 | 1 - 48 |
| 2 | Polyallylamin | 0,1 - 5 | 20 - 100 | 1 - 48 | 0,1 - 5 | 75 - 150 | 1 - 48 |
| 3 | Polyethylenimin | 0,1 - 5 | 20 - 100 | 1 - 48 | 0,1 - 5 | 75 - 150 | 1 - 48 |
| 4 | Chitosan | 0,1 - 5 | 20 - 100 | 1 - 48 | 0,1 -5 | 75 - 150 | 1 - 48 |
| 5 | Hercosettartige Verbindungen | 0,1 - 5 | 20 - 100 | 1 - 48 | 0,1 - 5 | 75 - 150 | 1 - 48 |
| 6 | Polyaminostyrol | 0,1 - 5 | 20 - 100 | 1 - 48 | 0,1 - 5 | 75 - 150 | 1 - 48 |
| 7 | Proteine | 0,1 - 5 | 20 - 100 | 1 - 48 | 0,1 - 5 | 75 - 150 | 1 - 48 |
| 8 | Polyol | - | - | - | 0,1 - 5 | 75 - 150 | 1 - 48 |

### Beispiel 2.1: Umsetzung von 1.12 mit Dimethylaminopropylamin

Zu einer Lösung von 6,27 g Verbindung **I.12** in 50 mL THF, wird eine Lösung aus 0,78 g (7,7 mmol) Dimethylaminopropylamin in 10 mL THF bei 66° C zugetropft. Zur Vervollständigung der Reaktion wird die Reaktionslösung für weitere 72 h unter Rückfluß erhitzt. Nach Entfernen des Lösungsmittels, erhält man ein dunkelgelbes viskoses Öl.

### Herstellung der quaternären Ammoniumverbindung

7,51 g (6 mmol) des obigen Produktes werden in 25 mL THF gelöst und bei RT eine Lösung aus 1,7 g (12 mmol) Mel in 5 mL THF zugetropft. Zur Reaktionsvervollständigung wird die Lösung weitere 3 h bei RT gerührt. Das Lösungsmittel wird im Rotationsverdampfer abdestilliert und der Rückstand mit 250 mL Et₂O gewaschen und im Hochvakuum über Nacht getrocknet. Das Produkt ist ein brauner, hygroskopischer Feststoff. Ausbeute: quantitativ.

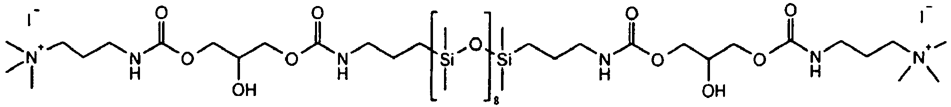

### Beispiel 2.2: Umsetzung von I.13 mit Dimethylaminopropylamin

Die Umsetzung und Quaternierung erfolgten analog zu Beispiel 2.1.

### Beispiel 2.3: Umsetzung von 1.1 mit Nonadimethylsiloxandipropylamin

Zu einer Lösung von 1,04 g (4 mmol) Verbindung **I.1** in 10 mL THF wird eine Lösung aus 1,62 g (2 mmol) Nonadimethylsiloxandipropylamin in 10 mL THF zugetropft und die Lösung für 96 h unter Rückfluss bei 66° C erhitzt. Nach der Entfernung des Lösungsmittels bleibt ein hochvisköses gelbes Öl zurück. Ausbeute: 2,5 g (2 mmol; 100% d. Theorie).

¹H-NMR (300 Mhz, CDCl₃):
δ_{H} = 0,03 (s, 6H, CH₃-17); 0,07 (s, br., 48H, CH₃-16); 0,53 (t, 4H, CH₂-15); 1,52 (m, 4H, CH₂-14); 1,66 (qu, 4H, CH₂-3); 2,20 (s, 12H, CH₃-1); 2,34 (t, 4H, CH₂-2); 3,18 (d, 8H, CH₂-4, CH₂-13); 3,70 (m, 2H, CH₂-8); 4,13 (m, 8H, CH₂-7, CH₂-10); 5,93 (s, br., 2H, NH) ppm.
¹³C- NMR- Spektrum (75 MHz, CDCl₃):
   δ_{C} = 1,05 (C-16, C-17, 2C); 15,23 (C-15, 2C); 23,82 (C-14, 2C); 26,96 (C-3, 2C); 30,32; 40,30 (C-2, 2C); 44,06 (C-13, 2C); 45,36 (C-1, 4C); 57,83 (C-4, 2C); 65,83; 156,88 (C-6, C-11, 4C) ppm.

### Herstellung der quaternären Ammoniumverbindung

3,3 g (2,6 mmol) der vorstehend erhaltenen Verbindung wird in 30 mL THF gelöst und bei RT eine Lösung aus 0,93 g (6,6 mmol) in 10 mL THF zugetropft. Der Ansatz wird für weitere 3 h bei RT gerührt. Dabei wird die Lösung trüb und nach einiger Zeit setzt sich eine farblose ölige Phase ab. Die THF-Phase wird abdekantiert und das Öl mehrmals mit THF gewaschen.

Zur Reinigung wird der Rückstand in CH₂Cl₂ gelöst und mit Et₂O ausgefällt. Das Produkt wird abfiltriert und über Nacht im HV getrocknet. Man erhält ein gelbliches, hygroskopisches Produkt, das unter N₂ gelagert wird. Ausbeute: quantitativ.
¹H-NMR (300 Mhz, DMSO-d₆):
δ_{H} = 0,03 (s, 6H, CH₃-17); 0,07 (s, br., 48H, CH₃-16); 0,48 (t, 4H, CH₂-15); 1,40 (m, 4H, CH₂-14); 1,84 (m, 4H, CH₂-3); 2,93 (m, 8H, CH₂-4, CH₂-13); 3,27 (m, 4H, CH₂-2); 3,92 (t, 8H, CH₂-7, CH-10, ³*J* = ); 4,74 (s, br., 1H, OH); 5,13 (s, br., 1H, OH); 7,17 (s, br., 2H, NH-12); 7,31 (s, br., 2H, NH-5) ppm.
   ¹³C- NMR- Spektrum (75 MHz, DMSO-d₆):
   δ_{C} = -0,65 - 0,33 (C-16, C-17, 18 C); 13,80 (C-15, 2C); 22,09 (C-14, 2C); 22,31 (C-3, 2C); 36,40 (C-4, 2C); 42,3 (C-13, 2C); 51,29 (C-1, 6C); 62,38 (C-2, 2C); 63,97 (C-10, 2C); 64,22 (C-7, 2C); 65,98 (C-8, 2C); 155,09 (C-11, 2C); 155,26 (C-6; 2C).

### Beispiel 2.4: Umsetzung von I.7 mit Nonadimethylsiloxandipropylamin

Die Umsetzung und Quaternierung erfolgten analog zu Beispiel 2.1.

### Beispiel 2.5: Umsetzung von 1.9 mit Dimethylaminopropylamin

Das mit einer C₁₂-Alkylkette funktionalisierte zyklische Carbonat (5.02 g, 15.2 mmol) wurde bei RT in CHCl₃ gelöst, 3-Dimethylaminopropylamin (2.22 g, 21.7 mmol) zugegeben und 2 Tage unter Rückfluss gerührt. Das Lösungsmittel und das überschüssige 3-Dimethylaminopropylamin wurden unter Vakuum entfernt. Das funktionelle Diurethan wurde ohne weitere Reinigung für die Quaternisierung der Aminogruppe eingesetzt.
(Ausbeute: quantitativ).

### Beispiel 2.6: Umsetzung von I.5 mit Dimethylaminopropylamin

Das mit einer C₁₈-Alkylkette funktionalisierte zyklische Carbonat (2.50 g, 6.04 mmol) und 3-Dimethylaminopropylamin (0.74 g, 7.25 mmol) wurden in 25 mL CHCl₃ gelöst und unter Rückfluss 20 Stunden gerührt. Das Lösungsmittel wurde im Vakuum entfernt und das funktionelle Diurethan wurde ohne weitere Reinigung für die Quaternisierung der Aminogruppe eingesetzt. (Ausbeute: quantitativ).

**Quaternisierung mit Mel:** Zu einer Lösung des tertiären Amins (5 mmol) in Aceton, Toluol oder Acetonitril (5 mL) wurde tropfenweise eine Lösung aus Mel (15 mmol) im selben Lösungsmittel (5 mL) zugegeben. Die Reaktion wurde bei RT 2 Stunden gerührt. Das Produkt fiel aus (eventuell mit Zugabe von Hexan als Fallungsmittel). Das Produkt wurde abfiltriert und getrocknet. (Ausbeute: 85-93%)

Quaternisierung mit n-Hexylbromid, C₁₂H₂₅-Br, n-C₁₈H₃₇-Br: Zu einer Lösung des tertiären Amins (5 mmol) in Acetonitril (10 mL) wurde das gewünschte Alkylbromid (n-Hexyl-, n-Dodecyl- oder Stearylbromid, 7 mmol) zugegeben. Die Reaktion wurde unter Rückfluss 22 Stunden gerührt. Das Lösungsmittel wurde unter Vakuum entfernt. Das Produkt wurde durch Austausch des Gegenions (Bromid gegen Chlorid) gereinigt (Quantitative Umsetzung des Amins).

Quaternisierung mit n-Hexyl-OTs, C₁₂H₂₅-OTs, n-C₁₈H₃₇-OTs:
Zu einer Lösung des tertiären Amins (5 mmol) in Acetonitril (10 mL) wurde das gewünschte Alkyltosylat (n-Hexyl-, n-Dodecyl- oder Stearyltosylat, 7 mmol) zugegeben. Die Reaktion wurde unter Rückfluss 20 Stunden gerührt. Das Lösungsmittel wurde unter Vakuum entfernt. Das Produkt wurde durch Austausch des Gegenions (Tosylat gegen Chlorid) gereinigt (Quantitative Umsetzung des Amins).

### Beispiel 3: Modifizierung von Polyaminen

### Beispiel 3.1: Modifizierung von Poly(allylamin)

Polyallylamin (M_{w}= -17000 g/mol) in 20 gew.% wässrige Lösung (1.00 g, 3.50 mmol) und eine Lösung der Verbindung **1.9** (288 mg, 0.875 mmol) in 4 mL THF wurden gemischt und 18 Stunden bei 80 °C gerührt. Nach Abkühlung war die Lösung klar. Nach Entfernung von THF unter Vakuum fiel das Produkt aus. NMR spektroskopische Analyse zeigte die Anwesenheit aller Bausteine im Produkt.

### Beispiel 3.2: Modifizierung von Poly(ethylenimin)

Polyethylenimin (M_{w}= 10000 g/mol, Aldrich) (15.2 mmol NH₂-Gruppen) und die Verbindung **1.3** (7.6 mmol) und Verbindung **I.10** (7.6 mmol) wurden in Methanol bei 70°C (oder alternativ in Dimethylformamid bei 80°C) 8h gerührt. Das Produkt wurde durch Ausfällen in Toluol isoliert. NMR spektroskopische Analyse zeigte die Anwesenheit aller Bausteine im Produkt.

### Beispiel 3.3: Modifizierung von Poly(dimethylsiloxan-co-methyl aminopropylsiloxan)

Zu einer Lösung aus 0,5 g (2,1 mmol) Verbindung A.1 in 5 mL THF wird eine Lösung aus 9,45 g (2,1 mmol) Poly-dimethylsiloxan-co-methylpropylaminsiloxan in 80 mL THF bei 0° C langsam zugetropft. Zur Reaktionsvervollständigung wird die Reaktionslösung für 72 h weiter gerührt. Dabei lässt man die Temperatur langsam auf Raumtemperatur steigen. Der Reaktionsansatz wird im Rotationsverdampfer eingeengt. Ausbeute: quantitativ

### Beispiel 4: Copolymerisation von 2-Oxo-1,3-dioxolan-4-yl-methylmethacrylat

### Beispiel 4.1: Copolymerisation von 2-Oxo-1,3-dioxolan-4-yl-methylmethacrylat und Methylmethacrylat

In einem ausgeheizten Kolben wurden unter einer Stickstoffatmosphäre 50 mmol Methylmethacrylat, 2,63 mmol 2-Oxo-1,3-dioxolan-4-yl-methylmethacrylat und 1,08 mmol Azoisobutyronitril in Methylethylketon vorgelegt. Die Copolymerisation wurde durch Eintauchen in ein auf 75 °C vorgeheiztes Ölbad gestartet. Nach 2,5 Stunden wurde die Umsetzung durch Eintauchen in ein Eisbad abgebrochen. Man versetzte das Reaktionsgemisch mit Dichlormethan, rührte 1 Stunde und fällte das gebildete Polymer mit n-Pentan. Das Polymer wurde 24 Stunden im Vakuumtrockenschrank bei 50 °C getrocknet. Ausbeute 90 % d. Th.; Mₙ (GPC) 13600; M_{w}/Nₙ = 7,8.

Das so hergestellte Polymer wurde mit Dodecylamin, 3-Dimethylamino-1-propylamin sowie mit einer Mischung beider Amine umgesetzt.

Polymeranaloge Umsetzung mit Dodecylamin:
In einem ausgeheizten Schlenkrohr wurden unter einer Stickstoffatmosphäre 300 mg Copolymer (dies entspricht 2,876 mmol an Wiederholungseinheiten und somit sind 0,144 mmol Wiederholungseinheiten polymeranalog umsetzbar) und 0,719 mmol Dodecylamin in Tetrahydrofuran vorgelegt. Es wurde bei RT gerührt. Nach 24 h wurde in Pentan gefällt. Der Rückstand wurde in Methylenchlorid aufgenommen und dreimal mit 1 molarer Natronlauge extrahiert. Die organische Phase wurde wieder in Pentan gefällt und 24 h im Vakuumtrockenschrank bei 50 °C getrocknet. Die Umsetzung erfolgte quantitativ.

Polymeranaloge Umsetzung mit 3-Dimethylamino-1-propylamin:
In einem ausgeheizten Schlenkrohr wurden unter einer Stickstoffatmosphäre 300 mg Copolymer (dies entspricht 2,876 mmol an Wiederholungseinheiten und somit sind 0,144 mmol Wiederholungseinheiten polymeranalog umsetzbar) und 0,719 mmol 3-Dimethylamino-1-propylamin in Tetrahydrofuran vorgelegt. Es wurde bei RT gerührt.
Nach 24 h wurde in Pentan gefällt. Der Rückstand wurde in Methylenchlorid auf- - genommen und dreimal mit 1 molarer Natronlauge extrahiert. Die organische Phase wurde wieder in Pentan gefällt und 24 h im Vakuumtrockenschrank bei 50 °C getrocknet. Die Umsetzung erfolgte quantitativ.

Polymeranaloge Umsetzung mit 3-Dimethylamino-1-propylamin und Dodecylamin (1:1):
In einem ausgeheizten Schlenkrohr wurden unter einer Stickstoffatmosphäre 300 mg Copolymer (dies entspricht 2,876 mmol an Wiederholungseinheiten und somit sind 0,144 mmol Wiederholungseinheiten polymeranalog umsetzbar), 0,072 mmol 3-Dimethylamino-1-propylamin und 0,072 mmol Dodecylamin in Tetrahydrofuran vorgelegt. Es wurde bei RT gerührt. Nach 24 h wurde in Pentan gefällt. Der Rückstand wurde in Methylenchlorid aufgenommen und dreimal mit 1 molarer Natronlauge extrahiert.
Die organische Phase wurde wieder in Pentan gefällt und 24 h im Vakuumtrockenschrank bei 50 °C getrocknet. Die Umsetzung erfolgte quantitativ.

### Beispiel 4.2: Copolymerisation von 2-Oxo-1,3-dioxolan-4-yl-methylmethacrylat, 2-Phenoxycarbonyloxy-ethylmethacrylat und Methylmethacrylat

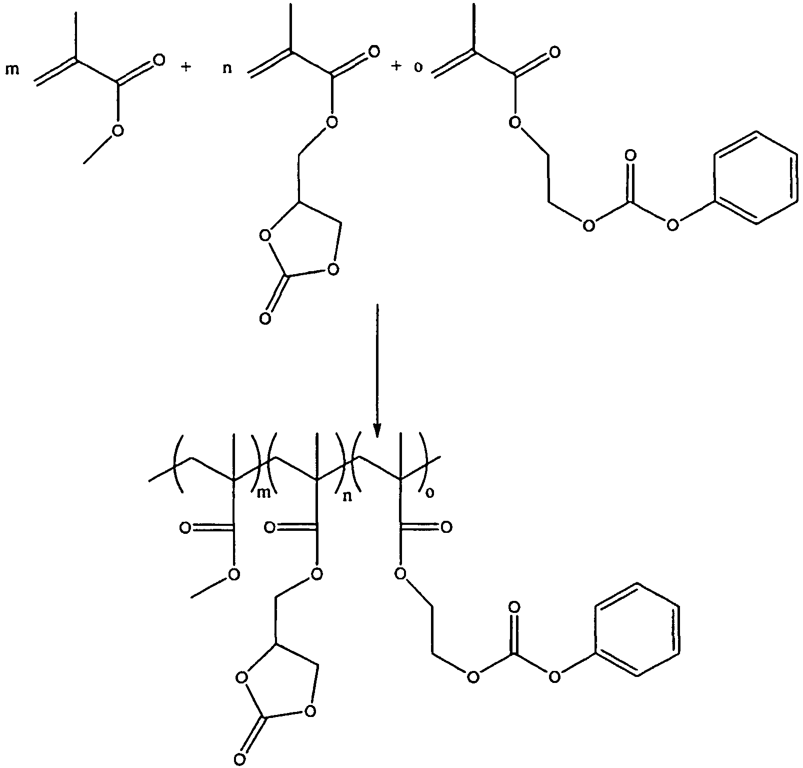

In einem ausgeheizten Kolben wurden unter einer Stickstoffatmosphäre 12 mmol Methylmethacrylat, 1,5 mmol 2-Oxo-1,3-dioxolan-4-yl-methylmethacrylat, 1,5 mmol 2-Phenoxycarbonyloxy-ethylmethacrylat (hergestellt durch Umsetzung von Hydroxyethylmethacrylat mit Phenylchloroformiat gemäß V. P. Joshi, S. K. Karode, M. G. Kulkarni, R. A. Mashelkar, Chem. Eng. Sci. 1998, 53, 2271) und 0,371 mmol Azoisobutyronitril in Methylethylketon vorgelegt. Die Copolymerisation wurde durch Eintauchen in ein auf 75 °C vorgeheiztes Ölbad gestartet. Nach 3 Stunden wurde die Umsetzung durch Eintauchen in ein Eisbad abgebrochen. Man versetzte das Reaktionsgemisch mit Dichlormethan, rührte 1 Stunde und fällte das gebildete Polymer mit n-Pentan. Das Polymer wurde 24 Stunden im Vakuumtrockenschrank bei 50 °C getrocknet. Ausbeute 85 % d. Th.; Mₙ (GPC) 9000; M_{w}/Nₙ = 2,9.

## Patentansprüche

1. Verfahren zur Modifizierung eines Substrates, das über funktionelle Gruppen verfügt, welche unter Hydroxygruppen, primären und sekundären Aminogruppen ausgewählt sind, bei dem man wenigstens ein Substrat unter Bedingungen mit einer Verbindung der Formel I oder II in Kontakt bringt, dass die funktionellen Gruppen unter Öffnung des 1, 3-Dioxolanrings bzw. 1,3-Diazaheptanrings und Ausbildung einer kovalenten Bindung mit der Verbindung der Formel I oder II reagieren, 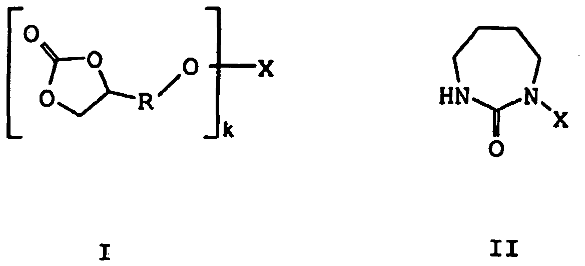 worin
R für C₁-C₁₂-Alkylen steht und k für 1 oder eine ganze Zahl von mehr als 1 steht;
wenn k für 1 steht, X für CO-CH=CH₂, CO-C(CH₃)=CH₂, CO-O-Aryl, C₂-C₆-Alkylen-SO₂-CH=CH₂, oder CO-NH-R¹ steht; und
R¹ für C₁-C₃₀-Alkyl, C₁-C₃₀-Halogenalkyl, C₁-C₃₀-Hydroxyalkyl, C₁-C₆-Alkyloxy-C₁-C₃₀-alkyl, C₁-C₆-alkylcarbonyloxy-C₁-C₃₀-alkyl, Amino-C₁-C₃₀-alkyl, Mono-oder Di(C₁-C₆-alkyl)amino-C₁-C₃₀-alkyl, Ammonio-C₁-C₃₀-alkyl, Polyoxyalkylen-C₁-C₃₀-alkyl, Polysiloxanyl-C₁-C₃₀-alkyl, (Meth)acryloyloxy-C₁-C₃₀-alkyl, Sultono-C₁-C₃₀-alkyl, Phosphono-C₁-C₃₀-alkyl, Di(C₁-C₆-alkyl)phosphono-C₁-C₃₀-alkyl, Phosphonato-C₁-C₃₀-alkyl, Di(C₁-C₆-alkyl)phosphonato-C₁-C₃₀-alkyl oder einen Saccharidrest steht, und
wenn k für eine ganze Zahl von mehr als 1 steht, X für (i) den Rest eines Polyamins, an das der in Klammern stehende Formelteil über (CO)NH-Gruppen gebunden ist, oder (ii) ein polymeres Gerüst steht, an das der in Klammern stehende Formelteil über (CO)-, NH-C₂-C₆-Alkylen-O(CO)- oder (CO)-O-C₂-C₆-Alkylen-O(CO)-Gruppen gebunden ist.

2. Verfahren nach Anspruch 1, wobei das Substrat unter Biomolekülen, Polymeren oder Oberflächen ausgewählt ist.

3. Verfahren nach Anspruch 2, wobei das Substrat ein Polymer ist.

4. Verfahren nach Anspruch 3, wobei in der Verbindung der Formel I oder II X für CO-NH-R¹ steht und zumindest ein Teil der Reste R¹ für Ammonioalkyl steht.

5. Verfahren nach Anspruch 4, wobei ein Teil der Reste R¹ für von Ammonioalkyl verschiedene Reste steht.

6. Verfahren nach Anspruch 1, wobei man die Verbindung der Formel 1 oder II mit einem ersten Substrat unter Bedingungen in Kontakt bringt, dass sich zwischen einem ersten Ende der Verbindung der Formel I oder II und dem ersten Substrat eine kovalente Bindung ausbildet, und das Reaktionsprodukt mit einem zweiten Substrat unter Bedingungen in Kontakt bringt, dass sich zwischen einem zweiten Ende der Verbindung der Formel I oder II und dem zweiten Substrat eine kovalente Bindung ausbildet.

7. Verfahren nach Anspruch 6, wobei das erste und/oder zweite Substrat unter Biomolekülen, Polymeren oder Oberflächen ausgewählt ist.

8. Verfahren nach Anspruch 7, wobei das Polymer ausgewählt ist unter Polyalkylenaminen, Polyvinylamin, Polyallylamin, Polyethylenimin, Chitosan, Polyamid-Epichlorhydrin-Harzen, Polyaminostyrol, Peptiden oder Proteinen.

9. Verfahren nach einem der vorhergehenden Ansprüche, wobei die Verbindung der Formel **I** ausgewählt ist unter
4-Phenyloxycarbonyloxymethyl-2-oxo-1,3-dioxolan,
4-(4-Phenyloxycarbonyloxy)butyl-2-oxo-1,3-dioxolan,
2-Oxo-1,3-dioxolan-4-yl-methylacrylat,
2-Oxo-1,3-dioxolan-4-yl-methylmethacrylat,
4-(2-Oxo-1,3-dioxolan-4-yl)-butylacrylat,
4-(2-Oxo-1,3-dioxolan-4-yl)-butylmethacrylat,
4-(Vinylsulfonylethyloxy)-butyl-2-oxo-1,3-dioxolan.

10. Verbindung der Formel I oder II, 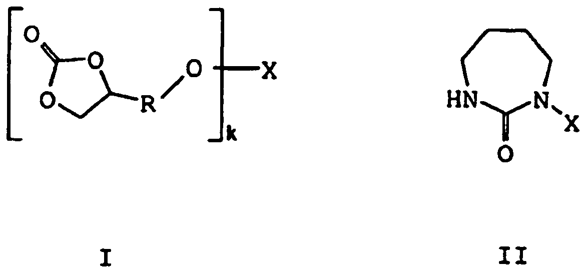 worin
R für C₁-C₁₂-Alkylen steht und k für 1 oder eine ganze Zahl von mehr als 1 steht;
wenn k für 1 steht, X für C₂-C₆-Alkylen-SO₂-CH=CH₂ oder CO-NH-R¹ steht; und
R¹ für C₁-C₃₀-Alkyl, C₁-C₃₀-Halogenalkyl, C₁-C₃₀-Hydroxyalkyl, C₁-C₆-Alkyloxy-C₁-C₃₀-alkyl, C₁-C₆-alkylcarbonyloxy-C₁-C₃₀-alkyl, Amino-C₁-C₃₀-alkyl, Mono-oder Di(C₁-C₆-alkyl)amino-C₁-C₃₀-alkyl, Ammonio-C₁-C₃₀-alkyl, Polyoxyalkylen-C₁-C₃₀-alkyl, Polysiloxanyl-C₁-C₃₀-alkyl, Sulfono-C₁-C₃₀-alkyl, Phosphono-C₁-C₃₀-alkyl, Di(C₁-C₆-alkyl)phosphono-C₁-C₃₀-alkyl, Phosphonato-C₁-C₃₀-alkyl, Di(C₁-C₆-alkyl)phosphonato-C₁-C₃₀-alkyl oder einen Saccharidrest steht, und
wenn R für C₂-C₁₂-Alkylen steht, X auch für CO-O-Aryl, CO-CH=CH₂, CO-C(CH₃)=CH₂ oder (Meth)acryloyloxy-C₁-C₃₀-alkyl-NH-CO stehen kann,
oder wenn k für eine ganze Zahl von mehr als 1 steht, X den Rest eines Polyamins steht, an das der in Klammern stehende Formelteil über (CO)NH-Gruppen gebunden ist, ausgenommen 4-[(2-Hydroxyethyl)-aminocarbonyloxymethyl]-2-oxo-1,3-dioxolan.

11. Verbindung nach Anspruch 10, worin R¹ für
- (CH₂)ₙ-CH₃,
- (CH₂)ₙ-(CF₂)ₘ-CF₃,
- (CH₂)ₙ-[Si(CH₃)₂-O]ₚ-H,
- (CH₂)ₙ-Si(OSi(CH₃)₃)₃,
- (CH₂)ₙ-(O-CH₂-CHR⁴)ₚ-OR³,
- R²-OH,
- R²-NH₂.
- R²-NR₃³⁺ Y⁻,
- R²-SO₃H,
- R²-PO₃H₂,
- R²-OPO₃H₂
oder einen Saccharidrest steht,
wobei R² für C₁-C₁₈-Alkylen, R³ für C₁-C₁₈-Alkyl oder Benzyl und R⁴ für Wasserstoff oder Methyl steht,
Y für ein Äquivalent eines Anions steht,
n und m unabhängig voneinander für eine ganze Zahl von 0 bis 12 stehen ; und
p für eine ganze Zahl von 1 bis 100 steht.

12. Verbindung nach Anspruch 10, ausgewählt unter 4-(4-Phenyloxycarbonyloxy)butyl-2-oxo-1,3-dioxolan,
2-Oxo-1,3-dioxolan-4-yl-methylacrylat,
2-Oxo-1,3-dioxolan-4-yl-methylmethacrylat,
4-(2-Oxo-1,3-dioxolan-4-yl)-butylacrylat,
4-(2-Oxo-1,3-dioxolan-4-yl)-butylmethacrylat,
4-(Vinylsulfonylethyloxy)-butyl-2-oxo-1,3-dioxolan.

13. Modifiziertes Polymer, erhältlich nach dem Verfahren nach einem der Ansprüche 3 bis 5.

14. Verwendung des modifizierten Polymers nach Anspruch 13 als Avivagemittel, Dispergator, Emulgator, Haftvermittler, Klebstoff, Haftklebstoff, zur Modifizierung von Oberflächen oder zur Immobilisierung von Wirkstoffen.

## Claims

1. A method for modifying a substrate which has functional groups selected from among hydroxyl groups, primary and secondary amino groups, in which method at least one substrate is brought into contact with a compound of formula I or II under conditions such that the functional groups react with the compound of formula I or II with opening of the 1,3-dioxolane ring or 1,3-diazaheptane ring and formation of a covalent bond, 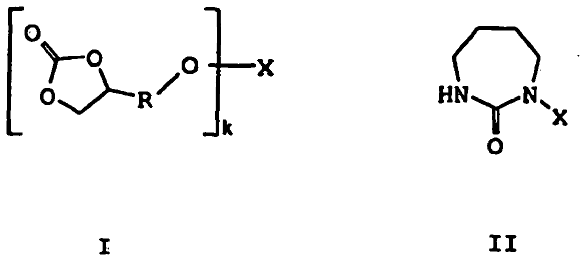 in which
R denotes C₁-C₁₂ alkylene and k denotes 1 or an integer of more than 1; if k denotes 1, X denotes CO-CH=CH₂, CO-C(CH₃)=CH₂, CO-O-aryl, C₂-C₆-alkylene-SO₂-CH=CH₂, or CO-NH-R¹; and
R¹ denotes C₁-C₃₀ alkyl, C₁-C₃₀ haloalkyl, C₁-C₃₀ hydroxyalkyl, C₁-C₆-alkyloxy-C₁-C₃₀-alkyl, C₁-C₆-alkylcarbonyloxy-C₁-C₃₀-alkyl, amino-C₁-C₃₀-alkyl, mono- or di(C₁-C₆-alkyl)amino-C₁-C₃₀-alkyl, ammonio-C₁-C₃₀-alkyl, polyoxyalkylene-C₁-C₃₀-alkyl, polysiloxanyl-C₁-C₃₀-alkyl, (meth)acryloyloxy-C₁-C₃₀-alkyl, sulfono-C₁-C₃₀-alkyl, phosphono-C₁-C₃₀-alkyl, di(C₁-C₆-alkyl)phosphono-C₁-C₃₀-alkyl, phosphonato-C₁-C₃₀-alkyl, di(C₁-C₆-alkyl)phosphonato-C₁-C₃₀-alkyl or a saccharide residue, and
if k denotes an integer of more than 1, X denotes (i) the residue of a polyamine, onto which the portion of the formula located between parentheses is attached via (CO)NH groups, or (ii) is a polymeric scaffold, onto which the portion of the formula located between parentheses is attached via (CO)-, NH-C₂-C₆-alkylene-O(CO) or (CO)-O-C₂-C₆-alkylene-O(CO) groups.

2. A method according to claim 1, wherein the substrate is selected from among biomolecules, polymers or surfaces.

3. A method according to claim 2, wherein the substrate is a polymer.

4. A method according to claim 3, wherein in the compound of formula I or II X denotes CO-NH-R¹ and at least a proportion of the residues R¹ denotes ammonioalkyl.

5. A method according to claim 4, wherein a proportion of the residues R¹ denotes residues other than ammonioalkyl.

6. A method according to claim 1, wherein the compound of formula 1 or II is brought into contact with a first substrate under such conditions that a covalent bond forms between a first end of the compound of formula I or II and the first substrate, and the reaction product is brought into contact with a second substrate under such conditions that a covalent bond forms between a second end of the compound of formula I or II and the second substrate.

7. A method according to claim 6, wherein the first and/or second substrate is selected from among biomolecules, polymers, or surfaces.

8. A method according to claim 7, wherein the polymer is selected from among polyalkyleneamines, polyvinylamine, polyallylamine, polyethyleneimine, chitosan, polyamide-epichlorohydrin resins, polyaminostyrene, peptides or proteins.

9. A method according to any one of the preceding claims, wherein the compound of formula I is selected from among
4-phenyloxycarbonyloxymethyl-2-oxo-1,3-dioxolane,
4-(4-phenyloxycarbonyloxy)butyl-2-oxo-1,3-dioxolane,
2-oxo-1,3-dioxolan-4-yl-methyl acrylate,
2-oxo-1,3-dioxolan-4-yl-methyl methacrylate,
4-(2-oxo-1,3-dioxolan-4-yl)-butyl acrylate,
4-(2-oxo-1,3-dioxolan-4-yl)-butyl methacrylate,
4-(vinylsulfonylethyloxy)-butyl-2-oxo-1,3-dioxolane.

10. A compound of formula I or II, 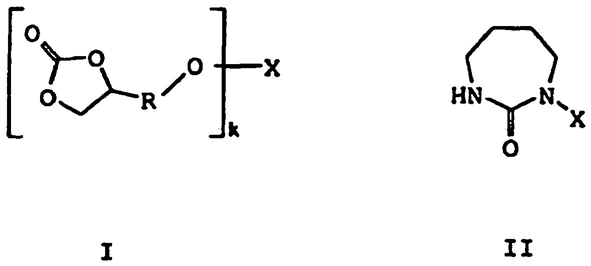 in which
R denotes C₁-C₁₂ alkylene and k denotes 1 or an integer of more than 1; if k denotes 1, X denotes C₂-C₆-alkylene-SO₂-CH=CH₂ or CO-NH-R¹; and R¹ denotes C₁-C₃₀ alkyl, C₁-C₃₀ haloalkyl, C₁-C₃₀ hydroxyalkyl, C₁-C₆-alkyloxy-C₁-C₃₀-alkyl, C₁-C₆-alkylcarbonyloxy-C₁-C₃₀-alkyl, amino-C₁-C₃₀-alkyl, mono- or di(C₁-C₆-alkyl)amino-C₁-C₃₀-alkyl, ammonio-C₁-C₃₀-alkyl, polyoxyalkylene-C₁-C₃₀-alkyl, polysiloxanyl-C₁-C₃₀-alkyl, sulfono-C₁-C₃₀-alkyl, phosphono-C₁-C₃₀-alkyl, di(C₁-C₆-alkyl)phosphono-C₁-C₃₀-alkyl, phosphonato-C₁-C₃₀-alkyl, di(C₁-C₆-alkyl)phosphonato-C₁-C₃₀-alkyl or a saccharide residue, and
if R denotes C₂-C₁₂ alkylene, X may also denote CO-O-aryl, CO-CH=CH₂, CO-C(CH₃)=CH₂ or (meth)acryloyloxy-C₁-C₃₀-alkyl-NH-CO, or if k denotes an integer of more than 1, X denotes the residue of a polyamine, onto which the portion of the formula located between parentheses is attached via (CO)NH groups, with the exception of 4-[(2-hydroxyethyl)-aminocarbonyloxymethyl]-2-oxo-1,3-dioxolane.

11. A compound according to claim 10, in which R¹ denotes
- (CH₂)ₙ-CH₃,
- (CH₂)ₙ-(CF₂)ₘ-CF₃,
- (CH₂)ₙ-[Si(CH₃)₂-O]ₚ-H,
- (CH₂)ₙ-Si(OSi(CH₃)₃)₃,
- (CH₂)ₙ-(O-CH₂-CHR⁴)ₚ-OR³,
- R²-OH,
- R²-NH₂,
- R²-NR₃³⁺ Y⁻,
- R²-SO₃H,
- R²-PO₃H₂,
- R²-OPO₃H₂
or a saccharide residue,
wherein R² denotes C₁-C₁₈ alkylene, R³ denotes C₁-C₁₈ alkyl or benzyl and R⁴ denotes hydrogen or methyl,
Y denotes an equivalent of an anion,
n and m in each case mutually independently denote an integer from 0 to 12;
and p denotes an integer from 1 to 100.

12. A compound according to claim 10, selected from among 4-(4-phenyloxycarbonyloxy)butyl-2-oxo-1,3-dioxolane,
2-oxo-1,3-dioxolan-4-yl-methyl acrylate,
2-oxo-1,3-dioxolan-4-yl-methyl methacrylate,
4-(2-oxo-1,3-dioxolan-4-yl)-butyl acrylate,
4-(2-oxo-1,3-dioxolan-4-yl)-butyl methacrylate,
4-(vinylsulfonylethyloxy)-butyl-2-oxo-1,3-dioxolane.

13. A modified polymer, obtainable by a method according to any one of claims 3 to 5.

14. Use of the modified polymer according to claim 13 as a finishing agent, dispersant, emulsifier, coupling agent, adhesive, pressure-sensitive adhesive, for modifying surfaces or for immobilising active ingredients.

## Revendications

1. Procédé pour la modification d'un substrat qui dispose de groupes fonctionnels qui sont choisis parmi les groupes hydroxy, les groupes amino primaires et secondaires, dans lequel on met en contact au moins un substrat avec un composé de formule I ou II dans des conditions telles que les groupes fonctionnels réagissent, avec ouverture du cycle 1,3-dioxolane ou, selon le cas, du cycle 1,3-diazaheptane et formation d'une liaison covalente avec le composé de formule I ou II, 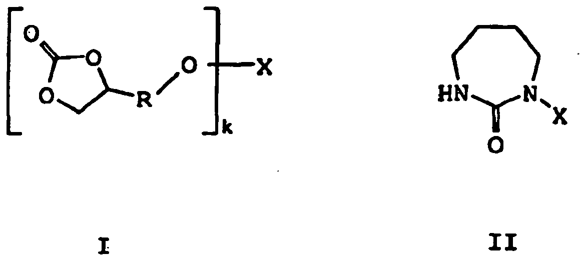 où
R représente C₁-C₁₂-alkylène et k vaut 1 ou un nombre supérieur à 1 ; lorsque k représente 1, X représente CO-CH=CH₂, CO-C(CH₃)=CH₂, CO-O-aryle, C₂-C₆-alkylène-SO₂-CH=CH₂ ou CO-NH-R¹ ; et
R¹ représente C₁-C₃₀-alkyle, C₁-C₃₀-halogénoalkyle, C₁-C₃₀-hydroxyalkyle, C₁-C₆-alkyloxy-C₁-C₃₀-alkyle, C₁-C₆-alkylcarbonyloxy-C₁-C₃₀-alkyle, amino-C₁-C₃₀-alkyle, mono-(C₁-C₆-alkyl)amino-C₁-C₃₀-alkyle ou di(C₁-C₆-alkyl)amino-C₁-C₃₀-alkyle, ammonio-C₁-C₃₀-alkyle, polyoxyalkylène-C₁-C₃₀-alkyle, polysiloxanyl-C₁-C₃₀-alkyle, (méth)acryloyloxy-C₁-C₃₀-alkyle, sulfono-C₁-C₃₀-alkyle, phosphono-C₁-C₃₀-alkyle, di(C₁-C₆-alkyl)phosphono-C₁-C₃₀-alkyle, phosphonato-C₁-C₃₀-alkyle, di(C₁-C₆-alkyl)phosphonato-C₁-C₃₀-alkyle ou un radical saccharide, et lorsque k vaut un nombre entier supérieur à 1, X représente (i) le radical d'une polyamine, auquel est liée la partie de formule se trouvant entre crochets via des groupes (CO)NH, ou (ii) une structure polymère, à laquelle est liée la partie de formule se trouvant entre crochets via des groupes (CO)-, NH-C₂-C₆-alkylène-O(CO)- ou (CO)-O-C₂-C₆-alkylène-O(CO)-.

2. Procédé selon la revendication 1, le substrat étant choisi parmi les biomolécules, les polymères ou les surfaces.

3. Procédé selon la revendication 2, le substrat étant un polymère.

4. Procédé selon la revendication 3, X représentant CO-NH-R¹ et au moins une partie des radicaux R¹ représentant ammonioalkyle dans le composé de formule I ou II.

5. Procédé selon la revendication 4, une partie des radicaux R¹ représentant des radicaux différents d'ammonioalkyle.

6. Procédé selon la revendication 1, le composé de formule I ou II étant mis en contact avec un premier substrat dans des conditions telles qu'il se forme une liaison covalente entre une première extrémité du composé de formule I ou II et le premier substrat et le produit de réaction étant mis en contact avec un deuxième substrat dans des conditions telles qu'il se forme une liaison covalente entre une deuxième extrémité du composé de formule I ou II et le deuxième substrat.

7. Procédé selon la revendication 6, le premier et/ou le deuxième substrat étant choisi parmi les biomolécules, les polymères ou les surfaces.

8. Procédé selon la revendication 7, le polymère étant choisi parmi les polyalkylène-amines, la polyvinylamine, la polyallylamine, la polyéthylène-imine, le chitosane, les résines de polyamide-épichlorhydrine, le polyaminostyrène, les peptides ou les protéines.

9. Procédé selon l'une quelconque des revendications précédentes, le composé de formule I étant choisi parmi
le 4-phényloxycarbonyloxyméthyl-2-oxo-1,3-dioxolane, le 4-(4-phényloxycarbonyloxy)butyl-2-oxo-1,3-dioxolane, l'acrylate de 2-oxo-1,3-dioxolan-4-ylméthyle, le méthacrylate de 2-oxo-1,3-dioxolan-4-ylméthyle, l'acrylate de 4-(2-oxo-1,3-dioxolan-4-yl)-butyle, le méthacrylate de 4-(2-oxo-1,3-dioxolan-4-yl)-butyle, le 4-(vinylsulfonyléthyloxy)-butyl-2-oxo-1,3-dioxolane.

10. Composé de formule I ou II, 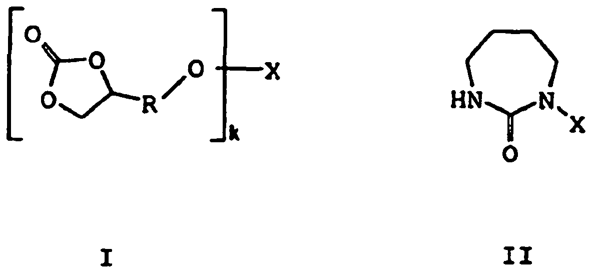 où
R représente C₁-C₁₂-alkylène et k vaut 1 ou un nombre entier supérieur à 1 ; lorsque k vaut 1, X représente C₂-C₆-alkylène-SO₂-CH=CH₂ ou CO-NH-R¹ ; et
R¹ représente C₁-C₃₀-alkyle, C₁-C₃₀-halogénoalkyle, C₁-C₃₀-hydroxyalkyle, C₁-C₆-alkyloxy-C₁-C₃₀-alkyle, C₁-C₆-alkylcarbonyloxy-C₁-C₃₀-alkyle, amino-C₁-C₃₀-alkyle, mono-(C₁-C₆-alkyl)amino-C₁-C₃₀-alkyle ou di(C₁-C₆-alkyl)amino-C₁-C₃₀-alkyle, ammonio-C₁-C₃₀-alkyle, polyoxyalkylène-C₁-C₃₀-alkyle, polysiloxanyl-C₁-C₃₀-alkyle, sulfono-C₁-C₃₀-alkyle, phosphono-C₁-C₃₀-alkyle, di(C₁-C₆-alkyl)phosphono-C₁-C₃₀-alkyle, phosphonato-C₁-C₃₀-alkyle, di(C₁-C₆-alkyl)phosphonato-C₁-C₃₀-alkyle ou un radical saccharide et, lorsque R représente C₂-C₁₂-alkylène, X peut également représenter CO-O-aryle, CO-CH=CH₂, CO-C(CH₃)=CH₂ ou (méth)acryloyloxy-C₁-C₃₀-alkyl-NH-CO, ou lorsque k vaut un nombre entier supérieur à 1, X représente le radical d'une polyamine, auquel la partie de formule se trouvant entre crochets est liée via des groupes (CO)NH- à l'exception du 4-[(2-hydroxyéthyl)-aminocarbonyloxyméthyl]-2-oxo-1,3-dioxolane.

11. Composé selon la revendication 10, R¹ représentant
- (CH₂)ₙ-CH₃,
- (CH₂)ₙ-(CF₂)ₘ-CF₃,
- (CH₂)ₙ-[Si(CH₃)₂-O]ₚ-H,
- (CH₂)ₙ-Si(OSi(CH₃)₃)₃,
- (CH₂)ₙ-(O-CH₂-CHR⁴)ₚ-OR³,
- R²-OH,
- R²-NH₂,
- R²-NR₃³⁺ Y⁻,
- R²-SO₃H,
- R²-PO₃H_{2,}
- R²-OPO₃H₂
ou un radical saccharide,
R² représentant C₁-C₁₈-alkylène, R³ représentant C₁-C₁₈-alkyle ou benzyle et R⁴ représentant hydrogène ou méthyle,
Y représentant un équivalent d'un anion,
n et m valant, indépendamment l'un de l'autre, un nombre entier de 0 à 12 ; et p valant un nombre entier de 1 à 100.

12. Composé selon la revendication 10, R¹ étant choisi parmi le 4-(4-phényloxycarbonyloxy)butyl-2-oxo-1,3-dioxolane,
l'acrylate de 2-oxo-1 ,3-dioxolan-4-ylméthyle,
le méthacrylate de 2-oxo-1,3-dioxolan-4-ylméthyle,
l'acrylate de 4-(2-oxo-1,3-dioxolan-4-yl)-butyle,
le méthacrylate de 4-(2-oxo-1,3-dioxolan-4-yl)-butyle,
le 4-(vinylsulfonyléthyloxy)-butyl-2-oxo-1,3-dioxolane.

13. Polymère modifié, pouvant être obtenu selon le procédé selon l'une quelconque des revendications 3 à 5.

14. Utilisation du polymère modifié selon la revendication 13 comme agent d'avivage, dispersant, émulsifiant, promoteur d'adhérence, adhésif, autoadhésif, pour la modification de surfaces ou pour l'immobilisation de substances actives.
